# EUROPEAN PATENT SPECIFICATION

(11) **EP 4 064 463 B1**
(45) Date of publication and mention of the grant of the patent: **13.05.2026**
(21) Application number: 20891030.7
(22) Date of filing: 20.11.2020
(51) Int. Cl.: H01R 13/518, A61J 15/00, A61M 31/00

(54) **CONNECTOR ASSEMBLY AND RETAINING DEVICE**
VERBINDERANORDNUNG UND HALTEVORRICHTUNG
ENSEMBLE CONNECTEUR ET DISPOSITIF DE RETENUE

(30) Priority: 20.11.2019 CN 201922030727 U
(43) Date of publication of application: 28.09.2022
(73) Proprietor: Fresenius Kabi (Nanchang) Co., Ltd., Nanchang, Jiangxi 330013 (CN)
(72) Inventor: XIA, Ningbo, Nanchang, Jiangxi 330013 (CN); XU, Jian, Nanchang, Jiangxi 330013 (CN); LAU, Fat Kit, Nanchang, Jiangxi 330013 (CN)
(74) Representative: Fresenius Kabi Deutschland GmbH
(86) International application number: PCT/CN2020/130357
(87) International publication number: WO 2021/098815

(56) References cited:
- WO-A1-2018/027944
- WO-A1-2019/200281
- CN-A- 105 688 309
- CN-U- 211 088 617
- US-A- 1 583 697
- US-A1- 2002 160 637
- US-A1- 2006 172 613
- US-A1- 2019 260 155
- US-B1- 6 350 155
- US-B1- 8 337 256

## Description

The present application claims priority to Chinese patent application No. 201922030727.5 filed with the China National Intellectual Property Administration on November 20, 2019 and titled "CONNECTOR ASSEMBLY AND HOLDING DEVICE".

### FIELD

The present disclosure relates to the field of medical apparatus, and in particular to a connector assembly for connecting a liquid infusion pump and a holding device and the holding device including the connector assembly.

### BACKGROUND

A liquid infusion pump is a kind of nutrition or pharmaceuticals infusion system commonly used in hospitals or other medical institutions, in which a pump tube and its accessories are connected through a nasogastric tube or a gastrointestinal tube, and an infusion speed, a dose and a total infusion amount, etc. are precisely controlled by a microcomputer. The liquid infusion pump is typically powered or signaled using a holding device. The liquid infusion pump and the holding device are usually electrically connected or signally connected through a pin connector assembly. The pin connector assembly includes a pin connector and a plastic housing surrounding the pin connector. The pin connector is provided with a ring of protruding ring for fixing the pin connector inside the plastic housing through an interference fit between the protruding ring and an inner wall of the plastic housing. However, in actual use, the pin connector is only held by the above-mentioned interference fit, and there is a risk of looseness between the pin connector and the plastic housing, so that the connection between the liquid infusion pump and the holding device has a risk of poor contact. US 8,337,256 A1 discloses a pogo-ping connector where spring biased pogo pins terminals can be moved from one position to the another, whereby contacting portions of conductive terminals abut against (conductive) stages of the pogo-pin terminals or against an insulative sleeve. The pogo-pin terminals are received in receiving pedestals which are used to seal at a bottom end of corresponding pogo-pin terminal slots of an insulating body, which are held in position by protrusions and a corresponding groove within the insulating body. The US 1,583,697 discloses an electrical connector comprising a pin within a two parted housing, shell and insulating body portion, wherein a spring is compressed to hold the pin outwardly of the shell when the insulating body portion is connected with the shell. Therefore, a plug portion is aligned with the insulating body. To connect the parts with each other a threaded connection is provided between the shell and the plug portion.

Therefore, there is a need for a connector assembly that enables a secure electrical or signal connection between the liquid infusion pump and the holding device.

### SUMMARY

The invention is defined by the appended claims.

An object of one or more embodiments of the present disclosure is to provide a connector assembly that enables a secure electrical or signal connection between a liquid infusion pump and a holding device.

Another object of one or more embodiments of the present disclosure is to provide a holding device including the connector assembly.

According to an aspect of the present disclosure, a connector assembly is provided, the connector assembly includes: a pin connector having one end which is configured to be electrically or signally connected with the liquid infusion pump and another end which is provided with a radially extending flange; a housing arranged around the pin connector to hold the pin connector, one end of the housing being provided with an annular step portion, the annular step portion and the flange of the pin connector cooperate to limit movement of the another end of the pin connector in one direction, wherein a top end of the step portion extends in a radially inward direction with a fixing portion which is pressed against the pin connector to limit the relative movement of the another end of the pin connector to the housing.

The pin connector includes a connecting pin located at the one end of the pin connector, the connecting pin is configured to be electrically or signally connected with the liquid infusion pump; an upper cover located at the another end of the pin connector which is configured to be electrically or signally connected with the holding device; a spring arranged between the connecting pin and the upper cover for applying a spring force and associated connection to the connecting pin to connect the liquid infusion pump and the holding device.

The upper cover includes an upper cover body and the flange extending radially outward from a top of the upper cover body.

A lower surface of the fixing portion is pressed against an upper surface of the flange to firmly fix the upper cover under the fixing portion of the housing.

The upper cover body is provided with a protruding ring in a radially outward direction, the protruding ring being caught on an inner circumferential surface of the housing to keep the upper cover from being ejected by the spring through an interference fit.

Preferably, the number of the pin connectors is two or five.

Preferably, each of the pin connectors is pressed by a plurality of the fixing portions.

Preferably, the fixing portions are evenly distributed along a periphery of the pin connector to provide uniform pressure to the pin connector.

A holding device is further provided according to another aspect of the present disclosure, the holding device is provided with the connector assembly as described above.

Preferably, a circuit board is arranged in the holding device, the circuit board being electrically or signally connected with the another end of the pin connector.

### BRIEF DESCRIPTION OF THE DRAWINGS

The features and advantages of one or more embodiments of the present disclosure will become more readily understood from the following description with reference to the drawings. In the drawings:
Figure 1 is a cross-sectional view showing a liquid infusion pump and a holding device according to a first embodiment of the present disclosure being connected.
Figure 2 is a partially enlarged view showing the connection portions of the liquid infusion pump and the holding device in Figure 1.
Figure 3 is a top view showing a connector assembly according to the first embodiment of the present disclosure.
Figure 4 is a front view showing the connector assembly according to the first embodiment of the present disclosure.
Figure 5 is a cross-sectional view showing the connector assembly taken along line A-A in Figure 4
Figure 6 is a partial enlarged view showing the connector assembly in Figure 5
Figure 7 is a cross-sectional view showing a connector assembly in a comparative example.
Figure 8 is a top view showing a connector assembly according to a second embodiment of the present disclosure.

### DETAILED DESCRIPTION OF EMBODIMENTS

The description of the preferred embodiments below is merely exemplary and is not intended to limit the present disclosure, its application or usage.

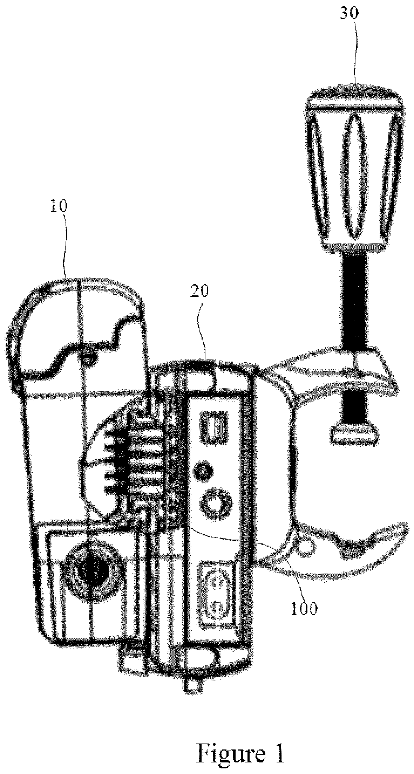

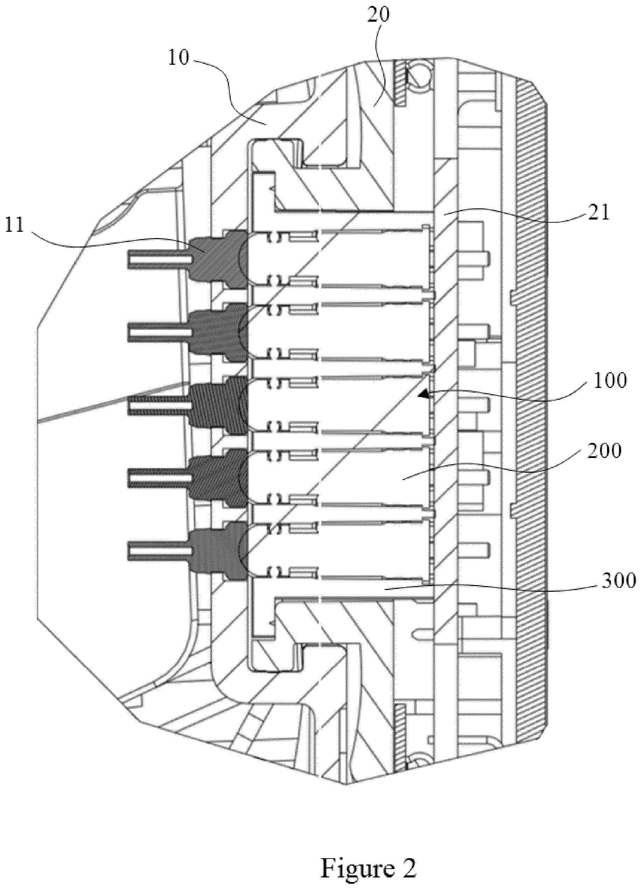

Figure 1 is a cross-sectional view showing a liquid infusion pump and a holding device according to a first embodiment of the present disclosure being connected. The liquid infusion pump 10 and the holding device 20 are detachably electrically or signally connected through a connector assembly 100. The holding device 20 may be fixed to the patient's bedside or an infusion stand by a threaded handle 30, thereby providing power to the liquid infusion pump 10 and communicating therewith while providing a mechanical support to the liquid infusion pump 10. Figure 2 is a partially enlarged view showing the connection portions of the liquid infusion pump 10 and the holding device 20 in Figure 1. Connection ports 11 are provided in the liquid infusion pump 10. A circuit board 21 is arranged in the holding device 20. The connector assembly 100 is arranged in the holding device 20. The connector assembly 100 includes five pin connectors 200 and a housing 300 that surrounds the pin connectors. One end of the connector 200 (the left end as shown in Figure 2) is electrically or signally connected to the connection port 11 in the liquid infusion pump 10. Another end of the pin connector 200 (the right end as shown in Figure 2) is electrically or signally connected to the circuit board 21 in the holding device 20.

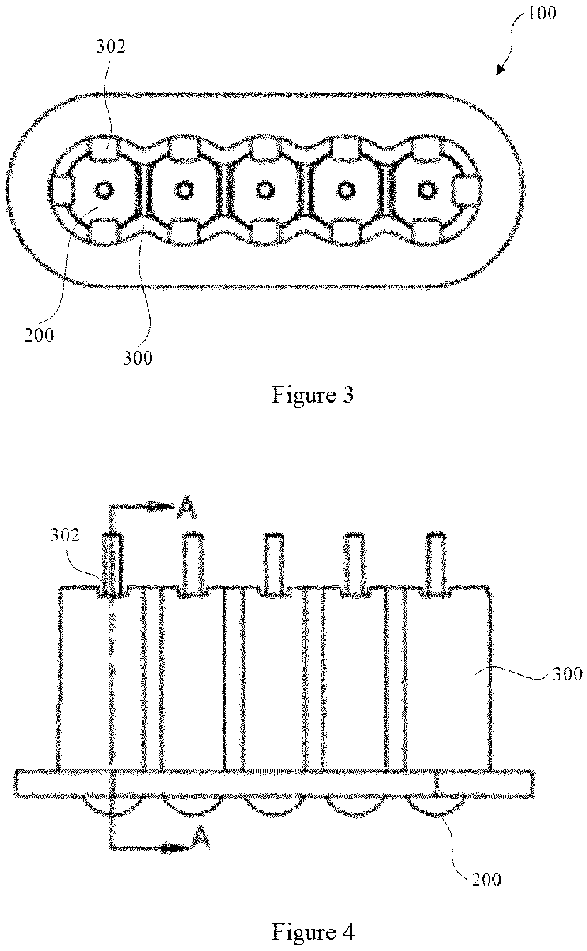

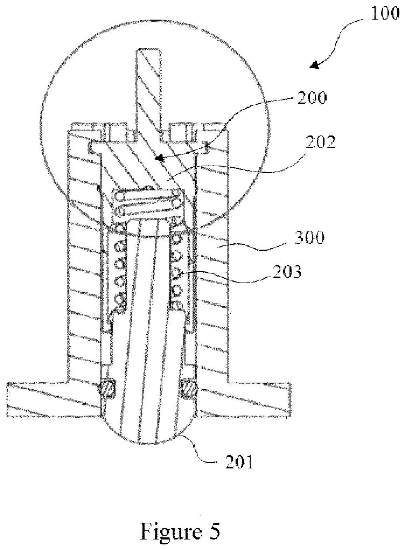

Figures 3 and 4 are top and front views showing the connector assembly 100 according to the first embodiment of the present disclosure, respectively. The pin connector assembly 100 is composed of five pin connectors 200 spaced apart by a distance and the housing 300 that surrounds the five pin connectors 200 to hold the pin connector 200. Preferably, the housing 300 is made of plastic. Figure 5 is a cross-sectional view showing the connector assembly 100 taken along line A-A in Figure 4. The pin connector 200 includes a connecting pin 201 which is configured to be electrically or signally connected with the connection port 11 of the liquid infusion pump 10, an upper cover 202 arranged at an end opposite to the connecting pin 201 which is configured to be electrically or signally connected with the circuit board 21 of the holding device 20, and a spring 203 arranged between the connecting pin 201 and the upper cover 202 for applying a spring force and connection between the connecting pin 201 and the upper cover 202, ensuring a reliable electrical or signal connection to the liquid infusion pump 10. Preferably, the upper cover 202 is made of metal. The housing 300 is arranged around the connecting pin 201, the upper cover 202, and the spring 203 to hold the connecting pin 201, the upper cover 202, and the spring 203.

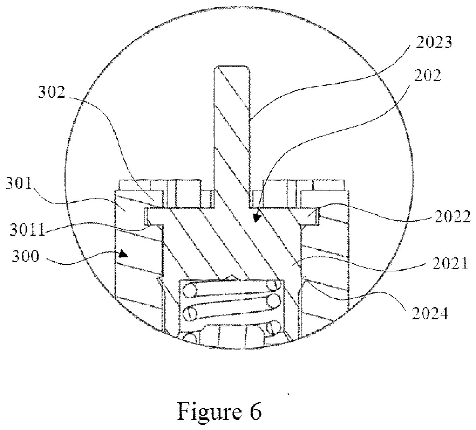

Figure 6 is a partial enlarged view showing the connector assembly 100 in Figure 5. The upper cover 202 includes an upper cover body 2021, a flange 2022 extending radially outward from a top end of the upper cover body 2021, and a rod portion 2023 arranged above the flange 2022. An upper portion of the housing 300 is provided with an annular step portion 301. A lower surface of the flange 2022 is pressed against a horizontal surface 3011 inside the step portion 301 to restrict a downward movement of the upper cover 202. A top end of the step portion 301 extends radially inwardly with at least one fixing portion 302, and a lower surface of the fixing portion 302 is pressed against an upper surface of the flange 2022 to securely fix the upper cover 202 inside the housing 300. Preferably, an area of an upper surface of the fixing portion 302 is smaller than an area of the upper surface of the remaining portion of the step portion 301, so that a portion of the step portion 301 of the housing 300 other than the fixing portion 302 may still be used to support and position the circuit board 21 (not shown) of the holding device 20. Preferably, as shown in Figure 4, each of the pin connectors 200 is pressed by a plurality of fixing portions 302. Preferably, as shown in Figure 4, the fixing portions 302 are evenly distributed along the periphery of the pin connector 200 to provide uniform pressure to the pin connector 200.

The fixing portion 302 of the connector assembly 100 according to the first embodiment of the present disclosure is realized by a processing process in which a plurality of small-area portions at the top end of the step portion 301 of the upper portion of the housing 300 are simultaneously melted by a heating tool such as a hot melt machine, and the melted portions form the radially inwardly extending fixing portions 302 which are pressed against the flange 2022 of the upper cover 202 to securely fix the upper cover 202 inside the housing 300.

In addition, as shown in Figure 6, the upper cover body 2021 is provided with a ring of continuous protruding ring 2024 in the radially outward direction. The protruding ring 2024 is caught in a corresponding groove portion on the inner circumferential surface of the housing 300 to keep the upper cover 202 from being ejected by the spring 203 through an interference fit.

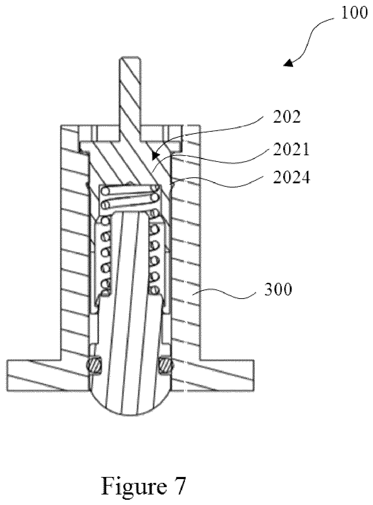

Figure 7 is a cross-sectional view showing a pin connector assembly 100 in a comparative example. In the comparative example, the connector assembly is provided with a ring of protruding ring 2024 only in the radially outward direction of the upper cover body 2021 of the upper cover 202. The protruding ring 2024 is caught in a groove on the inner circumferential surface of the housing 300 to keep the upper cover 202 from being ejected by the spring 203 through an interference fit. However, in actual use, the upper cover 202 is only held by the above-described interference fit, and there is a risk of looseness between the upper cover 202 and the plastic housing 300. When the upper cover 202 and the housing 300 are loosened, the spring 203 is in a relaxed state, and the connecting pin 201 is difficult to be ejected, and thus being easily disconnected from the connection port 11 of the pump 10, so that there is a risk of poor contact between the liquid infusion pump 10 and the holding device 20.

Compared with the pin connector assembly 100 in the comparative example of Figure 7, in the present disclosure, through the dual design having the fixing portion 302 and the protruding ring 2024, the upper cover 202 is firmly held in the housing 300 such that the spring 203 at the lower portion of the upper cover 202 is in a compressed state to provide the connecting pin 201 at the lower portion of the spring 203 with a spring force sufficient to eject it, so that the connecting pin 201 is firmly connected with the connection port 11 of the pump 10, thereby the liquid infusion pump 10 is firmly connected to the holding device 20.

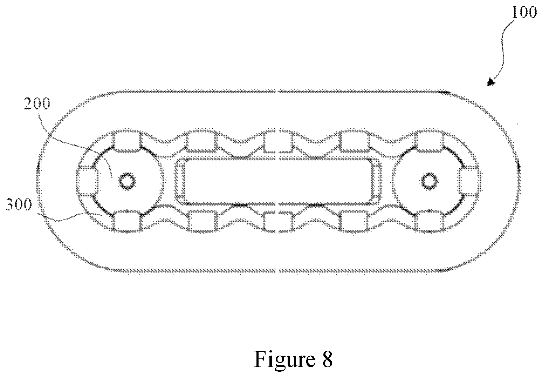

Figure 8 is a top view showing a connector assembly 100 according to a second embodiment of the present disclosure. The connector assembly 100 is composed of two pin connectors 200 and a housing 300 that surrounds the two pin connectors 200 to hold the pin connectors 200. The remaining structure of the connector assembly 200 in this embodiment is the same as the corresponding structure in the first embodiment. The connector assembly 100 may include a plurality of pin connectors 200 in an amount other than two or five in accordance with actual design needs.

Although the various embodiments of the present disclosure have been described in detail, it should be understood that the present disclosure is not limited to the specific embodiments described and illustrated in detail herein, and other variations and modifications may be made by those skilled in the art. The scope of the invention is defined by the appended claims.

## Claims

1. A connector assembly (100) for electrically or signally connecting a liquid infusion pump (10) and a holding device (20), such that the liquid infusion pump is powered or signaled using the holding device (20), with the connector assembly (100) comprising:
a pin connector (200) having one end which is configured to be electrically or signally connected with the liquid infusion pump (10) and another end which is provided with a radially extending flange (2022); comprising
a connecting pin (201) located at the one end of the pin connector (200), the connecting pin (201) being configured to be electrically or signally connected with the infusion pump (10), and
an upper cover (202) located at the another end of the pin connector (200) which is configured to be electrically or signally connected with the holding device (20), wherein the upper cover (202) comprises an upper cover body (2021) and the flange (2022) extending radially outward from a top of the upper cover body (2021), and
a spring (203) arranged between the connecting pin (201) and the upper cover (202) for applying a spring force and associated connection to the connecting pin (201);
a housing (300) arranged around the pin connector (200) to hold the pin connector (200), one end of the housing (300) being provided with an annular step portion (301), the annular step portion (301) and the flange (2022) of the pin connector (200) cooperate to limit movement of the another end of the pin connector (200) in one direction, **characterized in that**;
a lower surface of the flange (2022) is pressed against a horizontal surface (3011) inside the step portion (301) to restrict a downward movement of the upper cover (202) and a top end of the step portion (301) extends in a radially inward direction with a fixing portion (302) and a lower surface of the fixing portion (302) is pressed against an upper surface of the flange (2022) to limit the relative movement of the another end of the pin connector (200) to the housing (300), and the upper cover body (2021) is provided with a protruding ring (2024) in a radially outward direction, the protruding ring being caught in a groove on an inner circumferential surface of the housing (300).

2. The connector assembly (100) according claim 1, **characterized in that** the number of the pin connectors (200) is two or five.

3. The connector assembly (100) according to claim 2, **characterized in that** each of the pin connectors (200) is pressed by a plurality of the fixing portions (302).

4. The connector assembly (100) according to claim 3, **characterized in that** the fixing portions 302) are evenly distributed along a periphery of the pin connector (200).

5. A holding device (20), wherein the holding device (20) is provided with a connector assembly (100) as claimed in claims 1 to 4.

6. The holding device (20) according to claim 5, wherein a circuit board (21) is arranged in the holding device (20), the circuit board (21) being electrically or signally connected with the another end of the pin connector (200).

## Patentansprüche

1. Verbinderanordnung (100) zum elektrischen oder signalmäßigen Verbinden einer Flüssigkeitsinfusionspumpe (10) und einer Haltevorrichtung (20), so dass die Flüssigkeitsinfusionspumpe unter Verwendung der Haltevorrichtung (20) angetrieben oder signalisiert wird, wobei die Verbinderanordnung (100) Folgendes umfasst:
einen Stiftverbinder (200) mit einem Ende, das dazu konfiguriert ist, elektrisch oder signalmäßig mit der Flüssigkeitsinfusionspumpe (10) verbunden zu werden, und einem anderen Ende, das mit einem sich radial erstreckenden Flansch (2022) bereitgestellt ist; umfassend
einen Verbindungsstift (201), der sich an dem einen Ende des Stiftverbinders (200) befindet, wobei der Verbindungsstift (201) dazu konfiguriert ist, elektrisch oder signalmäßig mit der Infusionspumpe (10) verbunden zu werden, und
eine obere Abdeckung (202), die sich an dem anderen Ende des Stiftverbinders (200) befindet und dazu konfiguriert ist, elektrisch oder signalmäßig mit der Haltevorrichtung (20) verbunden zu werden, wobei die obere Abdeckung (202) einen oberen Abdeckungskörper (2021) und den Flansch (2022) umfasst, der sich von einer Oberseite des oberen Abdeckungskörpers (2021) radial nach außen erstreckt, und
eine Feder (203), die zwischen dem Verbindungsstift (201) und der oberen Abdeckung (202) angeordnet ist, um eine Federkraft und eine zugeordnete Verbindung auf den Verbindungsstift (201) auszuüben;
ein Gehäuse (300), das um den Stiftverbinder (200) herum angeordnet ist, um den Stiftverbinder (200) zu halten, wobei ein Ende des Gehäuses (300) mit einem ringförmigen Stufenabschnitt (301) bereitgestellt wird, wobei der ringförmige Stufenabschnitt (301) und der Flansch (2022) des Stiftverbinders (200) zusammenwirken, um die Bewegung des anderen Endes des Stiftverbinders (200) in einer Richtung zu begrenzen, **dadurch gekennzeichnet, dass**;
eine untere Fläche des Flansches (2022) gegen eine horizontale Fläche (3011) innerhalb des Stufenabschnitts (301) gepresst wird, um eine Abwärtsbewegung der oberen Abdeckung (202) zu begrenzen, und ein oberes Ende des Stufenabschnitts (301) sich in einer radial einwärts gerichteten Richtung mit einem Fixierabschnitt (302) erstreckt und eine untere Fläche des Fixierabschnitts (302) gegen eine obere Fläche des Flansches (2022) gepresst wird, um die relative Bewegung des anderen Endes des Stiftverbinders (200) zum Gehäuse (300) zu begrenzen, und der obere Abdeckkörper (2021) mit einem vorstehenden Ring (2024) in einer radial nach außen gerichteten Richtung bereitgestellt wird, wobei der vorstehende Ring in einer Nut auf einer inneren Umfangsfläche des Gehäuses (300) eingefangen wird.

2. Verbinderanordnung (100) nach Anspruch 1, **dadurch gekennzeichnet, dass** die Anzahl der Stiftverbinder (200) zwei oder fünf beträgt.

3. Verbinderanordnung (100) nach Anspruch 2, **dadurch gekennzeichnet, dass** jeder der Stiftverbinder (200) durch eine Vielzahl der Fixierabschnitte (302) gepresst wird.

4. Verbinderanordnung (100) nach Anspruch 3, **dadurch gekennzeichnet, dass** die Fixierabschnitte (302) gleichmäßig entlang eines Umfangs des Stiftverbinders (200) verteilt sind.

5. Haltevorrichtung (20), wobei die Haltevorrichtung (20) mit einer Verbinderanordnung (100) nach Anspruch 1 bis 4 bereitgestellt ist.

6. Haltevorrichtung (20) nach Anspruch 5, wobei eine Leiterplatte (21) in der Haltevorrichtung (20) angeordnet ist, wobei die Leiterplatte (21) mit dem anderen Ende des Stiftverbinders (200) elektrisch oder signalmäßig verbunden ist.

## Revendications

1. Ensemble connecteur (100) permettant de connecter électriquement ou par signaux une pompe à perfusion liquide (10) et un dispositif de maintien (20), de telle sorte que la pompe à perfusion liquide est alimentée ou reçoit des signaux à l'aide du dispositif de maintien (20), avec l'ensemble connecteur (100) comprenant :
un connecteur à broches (200) ayant une extrémité qui est conçue pour être connectée électriquement ou par signaux à la pompe à perfusion de liquide (10) et avec une autre extrémité qui est pourvue d'une bride s'étendant radialement (2022) ; comprenant
une broche de connexion (201) située au niveau de l'une extrémité du connecteur à broches (200), la broche de connexion (201) étant conçue pour être connectée électriquement ou par signaux à la pompe à perfusion (10), et
un couvercle supérieur (202) situé au niveau de l'autre extrémité du connecteur à broches (200) qui est conçu pour être connecté électriquement ou par signaux avec le dispositif de maintien (20), dans lequel le couvercle supérieur (202) comprend un corps de couvercle supérieur (2021) et la bride (2022) s'étendant radialement vers l'extérieur à partir d'un sommet du corps de couvercle supérieur (2021), et
un ressort (203) agencé entre la broche de connexion (201) et le couvercle supérieur (202) permettant d'appliquer une force de ressort et une connexion associée à la broche de connexion (201) ;
un boîtier (300) agencé autour du connecteur à broches (200) pour maintenir le connecteur à broches (200), une extrémité du boîtier (300) étant pourvue d'une partie étagée annulaire (301), la partie étagée annulaire (301) et la bride (2022) du connecteur à broches (200) coopèrent pour limiter le mouvement de l'autre extrémité du connecteur à broches (200) dans une direction, **caractérisé en ce que** ;
une surface inférieure de la bride (2022) est pressée contre une surface horizontale (3011) à l'intérieur de la partie étagée (301) pour limiter un mouvement vers le bas du couvercle supérieur (202) et une extrémité supérieure de la partie étagée (301) s'étend dans une direction radialement intérieure avec une partie de fixation (302) et une surface inférieure de la partie de fixation (302) est pressée contre une surface supérieure de la bride (2022) pour limiter le mouvement relatif de l'autre extrémité du connecteur à broches (200) par rapport au boîtier (300), et le corps du couvercle supérieur (2021) est pourvu d'un anneau en saillie (2024) dans une direction radiale vers l'extérieur, l'anneau en saillie étant engagé dans une rainure sur une surface circonférentielle interne du boîtier (300).

2. Ensemble connecteur (100) selon la revendication 1, **caractérisé en ce que** le nombre des connecteurs à broches (200) est de deux ou cinq.

3. Ensemble connecteur (100) selon la revendication 2, **caractérisé en ce que** chacun des connecteurs à broches (200) est pressé par une pluralité de parties de fixation (302).

4. Ensemble connecteur (100) selon la revendication 3, **caractérisé en ce que** les parties de fixation (302) sont réparties uniformément le long d'une périphérie du connecteur à broches (200).

5. Dispositif de maintien (20), dans lequel le dispositif de maintien (20) est pourvu d'un ensemble connecteur (100) tel que revendiqué dans les revendications 1 à 4.

6. Dispositif de maintien (20) selon la revendication 5, dans lequel une carte de circuit imprimé (21) est agencée dans le dispositif de maintien (20), la carte de circuit imprimé (21) étant connectée électriquement ou par signaux à l'autre extrémité du connecteur à broches (200).
